**Europäisches Patentamt**

**European Patent Office**

**Office européen des brevets**

(19)

(11) Veröffentlichungsnummer: **0 038 867**
**B1**

(12)

# EUROPÄISCHE PATENTSCHRIFT

(45) Veröffentlichungstag der Patentschrift:
28.09.83

(51) Int. Cl.³: **A 61 K 7/16**

(21) Anmeldenummer: 80102304.5

(22) Anmeldetag: 29.04.80

(54) Zahnpasta.

(43) Veröffentlichungstag der Anmeldung:
04.11.81 Patentblatt 81/44

(45) Bekanntmachung des Hinweises auf die Patenterteilung:
28.09.83 Patentblatt 83/39

(84) Benannte Vertragsstaaten:
AT BE CH DE FR GB IT LI LU NL SE

(56) Entgegenhaltungen:
DE-A-2 704 504
FR-A-2 096 701
FR-A-2 255 051
US-A-3 044 939
US-A-3 535 421
US-A-3 538 230
US-A-3 864 470
US-A-4 146 608
A ADR Abstracts 1975, Nr. 117

Die Akte enthält technische Angaben, die nach dem Eingang der Anmeldung eingereicht wurden und die nicht in dieser Patentschrift enthalten sind.

(73) Patentinhaber: **Blendax-Werke R. Schneider GmbH & Co., Rheinallee 88, D-6500 Mainz (DE)**

(72) Erfinder: **Becker, Dieter, Messeler-Park-Strasse 21, D-6100 Darmstadt-Wixhausen (DE)**
Erfinder: **Frosch, Franz, Dr., Eddersbacher Berg 6, D-6201 Taunusstein (DE)**
Erfinder: **Harth, Helmuth, Dr., Am Gonsenheimer Spiess 63, D-6500 Mainz (DE)**
Erfinder: **Raaf, Helmut, Dr., An der Schmalmach 1 B, D-6208 Bad Schwalbach (DE)**
Erfinder: **Wagner, Helmar R., Jägertorstrasse 108, D-6100 Darmstadt-Arheilgen (DE)**

# 0 038 867

## Zahnpasta

Die vorliegende Erfindung betrifft eine Zahnpasta, die eine ausgezeichnete zahnbelagsverhindernde Wirkung aufweist und damit einen wichtigen Beitrag zur Gesunderhaltung der Zähne und des Zahnfleisches leistet.

Daß Zahnbelag eine wesentliche Rolle sowohl bei der Entwicklung der Karies als auch der Parodontopathien spielt, ist heute in der zahnmedizinischen Wissenschaft unbestritten. Ein wesentlicher Teil der zahnmedizinischen Forschung ist deshalb darauf gerichtet, Substanzen und Mittel aufzufinden, die die Bildung von Zahnbelag auf den Zähnen zu verhindern vermögen. Dabei sind auch bereits wesentliche Fortschritte erzielt worden; eine der in dieser Hinsicht am besten untersuchten Verbindungen ist das unter dem Trivialnamen »Chlorhexidin« bekannte 1,6-Di-4'-(chlorphenyldiguanido)hexan, das nicht nur gegen die für die Bildung von Zahnbelag verantwortlichen Bakterien hervorragend wirkt, sondern auch auf den Zahnschmelz aufzieht und deshalb eine langdauernde Wirkung gewährleistet.

Leider weist das Chlorhexidin trotz seiner unbestreitbaren Erfolge insbesondere bei der Verhinderung bzw. Bekämpfung von Parodontopathien gewisse, an sich harmlose Nebenwirkungen auf, die bisher einer Daueranwendung in Zahn- und Mundpflegemitteln offenbar entgegenstanden:

Einerseits tritt beim längerem Gebrauch von Chlorhexidin enthaltenden Präparaten eine an sich harmlose Verfärbung der Zähne und der Schleimhäute auf, die zwar mechanisch beseitgt werden kann, trotzdem jedoch kosmetisch störend wirkt, darüberhinaus kann es auch zu Geschmacksirritationen kommen.

Es wurde deshalb versucht, Substanzen aufzufinden, die diese Nebenwirkungen nicht aufweisen, jedoch gleichwohl die Bildung von Zahnbelag wirksam verhindern können.

In diesem Zusammenhang ist auch bereits die Wirksamkeit von Kupferionen festgestellt worden, vgl. AADR Abstracts 1975, Nr. 117 (Journal of Dental research 1975, Spezial Issue A, S. 74).

Obwohl Kupferionen liefernde Verbindungen in Lösungen entsprechend der zitierten Vorveröffentlichung gute Resultate bei der Verhinderung von Zahnbelag ergeben haben, ist es überraschenderweise bislang nicht möglich gewesen, diese Resultate auch mit entsprechenden Zahnpasten zu erhalten.

Die vorliegende Erfindung geht daher von der Aufgabenstellung aus, eine Pasta mit einem Gehalt an gegen Zahnbelag wirksamen Kupferverbindungen zu entwickeln, die auch als Zahnpasta gegen die Bildung von Zahnbelag wirksam ist.

Die Lösung dieser Aufgabe besteht erfindungsgemäß darin, daß eine Zahnpasta auf Basis üblicher Grund- und Zusatzstoffe verwendet wird, die mindestens eine Kupferverbindung und mindestens ein Poliermittel enthält und dadurch gekennzeichnet ist, daß das Poliermittel zumindest zu mehr als der Hälfte aus einem Siliciumdioxid besteht.

Überraschenderweise zeigte sich nämlich, daß gerade bei der Verwendung dieses Poliermittels eine Zahnpasta mit einem Gehalt einer an sich bekannten Kupferverbindung eine hervorragende Wirksamkeit gegen Zahnbelag aufweist.

Bei den eingesetzten Siliciumdioxid-Poliermitteln handelt es sich um die an sich für diesen Zweck bereits seit längerem bekannten Produkte wie die durch Fällung erhaltene Kieselsäuren, wie sie beispielsweise in den DE-OS Nr. 2 206 285, 2 446 038 und 2 610 207 oder den GB-PSen Nr. 1 433 743 und 1 447 663 und der US-PS Nr. 4 122 160 beschrieben und beispielsweise unter den Bezeichnungen »Neosyl«® oder »Sident«® im Handel sind.

Ebenfalls ausgezeichnet geeignet für den erfindungsgemäßen Zweck sind die in der US-PS Nr. 3 538 230 beschriebenen Siliciumdioxid-Xerogele mit Oberflächen zwischen etwa 150 und etwa 800 m²/g, wie sie beispielsweise von der Firma Grace & Co. unter der Bezeichnung »Syloid«® vertrieben werden.

Auch teilweise dehydratisierte Siliciumdioxid-Hydrogele, wie sie aus den DE-OSen Nr. 2 704 504 und 2 920 906 bekannt sind, können in den erfindungsgemäßen Zahnpasten eingesetzt werden.

Die Teilchengrößen dieser Poliermittel liegen in der Regel in dem für diesen Zweck üblichen und optimalen Bereich, das heißt, zwischen etwa 1 und etwa 20 Mikron, vorzugsweise zwischen etwa 3 und etwa 14 Mikron.

Mit den oben näher beschriebenen Siliciumdioxid-Poliermitteln lassen sich wahlweise opake, transluzente oder auch transparente Zahnpasten herstellen, was vom Verhältnis Wasser zum Feuchthaltemittel (insbesondere Glycerin und/oder Sorbit) und der Abwesenheit gegebenenfalls opazifizierender Mittel abhängig ist, wodurch der hierfür maßgebliche Brechungsindex der Pastengrundlage bestimmt wird. Brechungsindices zwischen etwa 1,43 und 1,46 (20°C) führen zu transparenten Produkten.

Eine weitere Gruppe von im Rahmen der vorliegenden Erfindung einsetzbaren Siliciumdioxid-Poliermitteln sind kristalline Siliciumdioxide, wie sie beispielsweise in der DE-OS Nr. 2 036 551 beschrieben sind.

Eine ausführliche Übersicht über in den erfindungsgemäßen Zahnpasten verwendbare Siliciumdioxid-Typen gibt H. Ferch in seiner Arbeit in »Chemie—Ingenieur—Technik«, Band 48 (1976), S. 922—933, auf die hier im Rahmen der Offenbarung ausdrücklich Bezug genommen wird.

2

Vorzugsweise gelangt das Siliciumdioxid in der erfindungsgemäßen Zahnpasta als alleiniges Poliermittel zum Einsatz.

Es ist jedoch auch möglich, andere Poliermittel in untergeordneten Mengen (d. h., in jedem Fall weniger als die Hälfte des gesamten Poliermittels) einzusetzen, sofern diese nicht inaktivierend auf die vorhandenen Kupferverbindungen wirken.

Derartige Poliermittel sind beispielsweise pulverförmige Kunststoffe wie Polyvinylchlorid, Polyvinylfluorid, Polymethylmethacylat, Aminoplaste wie Harnstoff- oder Melamin-Formaldehyd-Kondensate mit Teilchengrößen zwischen etwa 0,5 und etwa 40 Mikron, vorzugsweise etwa 2 bis 20 Mikron.

Geeignete Kunststoff-Poliermittel sind beispielsweise in den US-PSen Nr. 2 130 034, 3 070 510, 3 251 800, 3 357 950 und 3 151 027 sowie der DE-AS Nr. 1 617 306 beschrieben.

Die Menge an Kupferverbindungen, die in den erfindungsgemäßen Zahnpasten zum Einsatz gelangt, sollte so bemessen sein, daß etwa 0,001 bis etwa 5 Gew.-% Cu, berechnet auf die gesamte Zahnpasta, anwesend sind.

Ein bevorzugter Mengenbereich liegt bei 0,05 bis 1,5%, insbesondere bis eta 0,5% Cu.

Als Kupferionen liefernde Kupferverbindungen sind prinzipell alle toxikologischen unbedenklichen, schleimhautverträglichen, auch nur einigermaßen wasserlöslichen Kupferverbindungen geeignet.

Von den anorganischen Kupfersalzen seien beispielhaft genannt:
Kupferchlorid, $CuCl_2$, und dessen Dihydrat; Kupferfluorid, $CuF_2$, und dessen Dihydrat; Kupferfluorsilikat, $CuSiF_6$, und dessen Hexahydrat; Kupfersulfat, $CuSO_4$, und dessen Pentahydrat; Kupfernitrat bzw. dessen Tri- und Hexahydrat sowie auch ausgefallenere Kupfersalze wie Kupferbromid, $CuBr_2$; Kupfermetaborat, $Cu(BO_2)_2$; Kupferbromat, $Cu(BrO_3)_2 \cdot 6 H_2O$; Kupferchlorat, $Cu(ClO_3)_2 \cdot 6 H_2O$; Kupferjodat, $Cu(IO_3)_2$, und Kupferfluorphosphat, $CuPO_3F$.

Bevorzugte Kupfersalze organischer Säuren sind Kupferacetat, Kupferformiat, Kupferbenzoat, Kupfercitrat, Kupfertartrat, Kupferlactat, Kupfermalat, Kupfermandelat, Kupfersorbat, Kupferpantothenat, Kupfergluconat, Kupferphytat, Kupferglycerophosphat, Kupfercinnamat, Kupferbutyrat, Kupferpropionat, Kupferlaurat, Kupferoxalat und Kupfersalicylat.

Wie bereits erwähnt, enthalten die erfindungsgemäßen Zahnpasten neben dem obligatorischen Siliciumdioxid-Poliermittel und einer oder mehreren Kupferverbindungen übliche Zusatz- und Grundstoffe.

Hier sind insbesondere die bekannten Feuchthaltemittel zu nennen, insbesondere Glycerin und andere Polyalkohole wie Propylenglycol, 1,3 Butandiol und Polyethylenglycole mit niederem Molgewicht sowie Zuckeralkohole, insbesondere Sorbit und gegebenenfalls auch Mannit und Xylit.

Darüberhinaus enthalten Zahnpasten üblicherweise Verdickungs- und Bindemittel. Im vorliegenden Fall eignen sich am besten Cellulosederivate, insbesondere Hydroxyalkylcellulose wie Hydroxyethylcellulose und Pflanzengummen, wie Xanthan Gum, Irish Moos und gegebenenfalls auch gegenüber Kupferionen inerte anorganische Verdickungsmittel, deren Anteil üblicherweise zwischen etwa 0,25 und etwa 3,5 Gew.-% der Zahnpasta beträgt.

Die erfindungsgemäßen Zahnpasten können auch oberflächenaktive Substanzen enthalten. Diese werden insbesondere zur Erzeugung einer vom Verbraucher geschätzten Schaumkraft eingesetzt. Geeignete oberflächenaktive Substanzen sind besonders wasserlösliche Salze von höheren Alkylsulfaten oder Alkyläthersulfaten, beispielsweise Natriumlaurylsulfat, aliphatische Acylamide gesättigter Monoaminocarbonsäuren, vorzugsweise Natrium-N-lauroylsarcosinat, Taurin-Fettsäureamide, beispielsweise Natrium-N-alkyl-N-myristoyltaurid, Salze von sulfonierten Monoglyceriden höherer Fettsäuren, beispielsweise Natriummonoglyceridsulfonat, Fettsäureester der Isäthionsäure und deren Salze, nichtionische Tenside wie Alkylenoxidkondensate mit Fettalkoholen und ein- oder mehrwertigen Aminen, Zuckerester, beispielsweise Saccharosemonolaurat, Sorbitolpolyoxyäthylenstearat, langkettige Aminoxide, beispielsweise Dimethyllaurylaminoxid, ampholytische Tenside, beispielsweise Betaine oder langkettige Alkylaminocarbonsäuren, und kationaktive Tenside, beispielsweise quartäre Ammoniumverbindungen wie Cetyltrimethylammoniumbromid.

Der Anteil an oberflächenaktiven Verbindungen in der erfindungsgemäßen Zahnpasta liegt bei 0 bis etwa 5 Gew.-% der Gesamtzusammensetzung. Zahnpasten enthalten üblicherweise Aroma- und Geschmackstoffe, Konservierungsmittel etc., derartige Mittel sind an sich bekannt und in zahlreichen Druckschriften beschrieben.

Es ist eine bevorzugte Ausführungsform der Erfindung, Fluorverbindungen in der erfindungsgemäßen Zahnpasta mitzuverwenden, vorzugsweise in solchen Mengen, daß die Konzentration an reinem Fluor im Mittel 0,01 bis 1 Gew.-%, vorzugsweise 0,1 bis 0,5 Gew.-% der Zahnpasta beträgt.

Geeignete Fluorverbindungen sind insbesondere die verschiedenen Salze der Monofluorphosphorsäure, insbesondere Natrium-, Kalium-, Lithium-, Calcium- und Aluminiummono- und difluorphosphate, sowie die verschiedenen, Fluor in ionisch gebundener Form enthaltenden Fluoride, insbesondere Alkalifluoride wie Natrium-, Lithium-, Kalium- und Ammoniumfluorid, Zinnfluorid, Manganfluorid, Zirkoniumfluorid und Aluminiumfluorid sowie Gemische der Anlagerungsprodukte dieser Fluoride untereinander oder mit anderen Fluorverbindungen, beispielsweise Kalium- oder Natriummanganfluorid.

Andere im Rahmen der vorliegenden Erfindung einsetzbare Fluoride sind beispielsweise Zinkfluorid,

3

**0 038 867**

Germaniumfluorid, Palladiumfluorid, Titanfluorid, Alkalifluorzirkonate, beispielsweise Natrium- oder Kaliumfluorzirkonat, Zinnfluorzirkonat, Fluorborate oder Fluorsulfate, beispielsweise Natrium- oder Kaliumfluorsulfat.

Fluor- oder Kupferionen können in der erfindungsgemäßen Zahnpasta auch durch eine Verbindung zur Verfügung gestellt werden, solche Verbindungen sind beispielsweise Kupferfluorid, Kupfermonofluorphosphat und Kupferfluorsilikat.

Auch organische Fluorverbindungen können mit Erfolg eingesetzt werden, insbesondere die bekannten Additionsprodukte aus langkettigen Aminen oder Aminosäuren und Fluorwasserstoff, Monoäthanolaminohydrofluorid oder Methyltriäthylammoniumfluorid.

Die erfindungsgemäßen Zahnpasten können weitere, zur Verwendung in solchen Mitteln an sich bekannten Stiffe enthalten, beispielsweise Enzyme wie Proteasen und Carbohydrasen, z. B. Amylase, Dextranase, Lävanase oder $\alpha$-1,3-Glucan-3-glucanohydrolase, Zahnstein entfernende Substanzen wie die für diesen Zweck vorgeschlagenen Phosphonsäuren, beispielsweise Hydroxyäthan-1,1-diphosphonsäure, oder die als Zahnbelag verhindernde Substanzen bekannten Bisbiguanide bzw. deren vorzugsweise wasserlösliche Salze.

Eine ausführliche Übersicht über die Herstellung von Zahnpflegemitteln und die dabei zum Einsatz gelangenden Stoffe findet sich in dem Handbuch von M. S. Balsam und E. Sagarin, »Cosmetics-Science and Technology«, 2nd Ed., Vol. 1, S. 423 bis 531 (1972).

Im folgenden werden einige Beispiele für erfindungsgemäß zusammengesetzte Zahnpasten gegeben. Bei den in den Beispielen 1, 2 und 3 beschriebenen Zahnpasten handelt es sich um opake, bei den in den Beipsielen 4, 5 und 6 beschriebenen Zahnpasten um transparente bzw. transluzente Produkte.

### Beispiel 1

| | |
|---|---|
| Xanthan Gum | 1,00 (Gew.-%) |
| Glycerin | 10,00 |
| Sorbit | 15,00 |
| $CuSO_4 \cdot 5 H_2O$ | 0,20 |
| Natriummonofluorphosphat | 0,76 |
| Natriumlaurylsulfat | 1,60 |
| Gefällte Kieselsäure (vom Typ Neosyl®) | 20,00 |
| Titandioxid | 0,70 |
| Aromagemisch | 1,00 |
| Saccharin-Natrium | 0,10 |
| p-Hydroxybenzoesäuremethylester | 0,20 |
| Entsalztes Wasser | 49,44 |

### Beispiel 2

| | |
|---|---|
| Irish Moos | 0,50 (Gew.-%) |
| Xanthan Gum | 0,50 |
| Glycerin | 7,50 |
| Sorbit | 28,00 |
| Kupferformiat $\cdot$ 4 $H_2O$ | 0,30 |
| Kupferfluorid ($CuF_2$) | 0,25 |
| Natriumlauroylsarcosinat | 1,40 |
| Gefällte Kieselsäure (vom Typ Sident®3) | 22,50 |
| Titandioxid | 0,50 |
| Saccharin-Natrium | 0,10 |
| Aromagemisch | 1,00 |
| p-Hydroxybenzoesäuremethylester | 0,10 |
| p-Hydroxybenzoesäure-n-propylester | 0,05 |
| Entsalztes Wasser | 37,30 |

### Beispiel 3

| | |
|---|---|
| Xanthan Gum | 1,20 (Gew.-%) |
| Glycerin | 15,00 |
| Sorbit | 12,00 |
| Kupfersalicylat ($Cu(C_7H_5O_3)_2 \cdot 4 H_2O$) | 1,00 |

4

| | |
|---|---|
| Natriumlaurylsulfat | 1,40 |
| Siliciumdioxid-Xerogel | |
| (vom Typ Syloid® AL 1, Oberfläche etwa 800 m²/g) | 16,00 |
| Pyrogenes Siliciumdioxid (vom Typ Aerosil®) | 3,00 |
| Titandioxid | 0,50 |
| Aromagemisch | 1,00 |
| Saccharin-Natrium | 0,16 |
| Trinatriumcitrat | 0,25 |
| p-Hydroxybenzoesäureäthylester | 0,20 |
| Entsalztes Wasser | 48,29 |

## Beispiel 4

| | |
|---|---|
| Hydroxyäthylcellulose | 1,10 (Gew.-%) |
| Glycerin | 26,00 |
| Sorbit | 23,50 |
| Kupferfluosilikat ($CuSiF_6 \cdot 6\ H_2O$) | 0,95 |
| Natriumlaurylsulfat | 1,40 |
| Siliciumdioxid-Xerogel | |
| (vom Typ Syloid® 74, Oberfläche etwa 290 m²/g) | 20,00 |
| Siliciumdioxid-Aerogel | |
| (Syloid® 244, Oberfläche etwa 260 m²/g) | 1,80 |
| Aromagemisch | 1,10 |
| Saccharin-Natrium | 0,15 |
| p-Hydroxybenzoesäuremethylester | 0,20 |
| Blaue Farbstofflösung (C.I.-Nr. 42 051); 1%ig | 0,05 |
| Alkoholischer Kräuterextrakt | 0,20 |
| Entsalztes Wasser | 23,55 |

## Beispiel 5

| | |
|---|---|
| Hydroxypropylcellulose | 1,20 (Gew.-%) |
| 1,3-Butandiol | 6,00 |
| Glycerin | 23,00 |
| Sorbit | 20,00 |
| Natriumlaurylsulfat | |
| (10%ige Aufschlämmung in Glycerin) | 8,50 |
| Kupferfluorid ($CuF_2 \cdot 2\ H_2O$) | 0,30 |
| Kupferpantothenat | 0,80 |
| Gefällte Kieselsäure (vom Typ Sident®3) | 20,00 |
| Polyäthylenglykol 600 | 2,00 |
| Aromagemisch | 1,20 |
| Saccharin-Natrium | 0,20 |
| Rote Farbstofflösung (C.I.-Nr. 16 255); 1%-ig | 0,07 |
| p-Hydroxybenzoesäureäthylester | 0,13 |
| Benzoesäure | 0,10 |
| Weinsäure | 0,55 |
| Entsalztes Wasser | 15,95 |

## Beispiel 6

| | |
|---|---|
| Xanthan Gum | 0,40 (Gew.-%) |
| Glycerin | 28,00 |
| Sorbit (70%ig) | 17,00 |
| Polyäthylenglykol 300 | 3,00 |
| Kupferlactatdihydrat | 1,20 |
| Zinnfluorid ($SnF_2$) | 0,40 |
| Hydroxyäthan-1,1-diphosphonsäure, Trinatriumsalz | 1,25 |
| Bromchlorophen | 0,05 |
| Benzoesäure | 0,15 |
| Dehydracetsäure | 0,10 |

| | |
|---|---|
| p-Hydroxybenzoesäure-n-propylester | 0,05 |
| Siliciumdioxid-Xerogel | |
| (vom Typ Syloid® 70, Oberfläche etwa 290 m²/g) | 23,50 |
| Pyrogenes Siliciumdioxid (vom Typ Aerosil®) | 1,20 |
| Natriumlaurylethersulfat (25%ig in Ethanol) | 10,00 |
| Entsalztes Wasser | 13,70 |

## Patentansprüche

1. Zahnpasta auf wäßriger Basis mit üblichen Grund- und Zusatzstoffen, enthaltend mindestens ein Poliermittel und 0,001-5 Gew.-%, bezogen auf die Gesamtzusammensetzung (berechnet als Cu), mindestens einer Kupferionen liefernden Verbindung, dadurch gekennzeichnet, daß das Poliermittel zu mehr als der Hälfte aus einem Siliciumdioxid besteht.

2. Zahnpasta nach Anspruch 1, dadurch gekennzeichnet, daß das Siliciumdioxid-Poliermittel zumindest teilweise aus einem gefällten, gegebenenfalls ganz oder teilweise dehydratisierten Siliciumdioxid-Gel besteht.

3. Zahnpasta nach Anspruch 1, dadurch gekennzeichnet, daß das Siliciumdioxid-Poliermittel zumindest teilweise aus einem Siliciumdioxid-Xerogel besteht.

4. Zahnpasta nach Anspruch 1, dadurch gekennzeichnet, daß das Siliciumdioxid-Poliermittel zumindest teilweise aus einem gegebenenfalls teilweise dehydratisierten, Siliciumdioxid-Hydrogel besteht.

5. Zahnpasta nach einem der vorhergehenden Ansprüche, dadurch gekennzeichnet, daß sie als Kupferionen liefernde Verbindung ein wasserlösliches anorganisches Kupfersalz enthält.

6. Zahnpasta nach einem der Ansprüche 1 bis 4, dadurch gekennzeichnet, daß sie als Kupferionen liefernde Verbindung ein Kupfersalz einer organischen Säure enthält.

7. Zahnpasta nach Anspruch 6, dadurch gekennzeichnet, daß sie als Kupfersalz einer organischen Säure mindestens eine der Verbindungen Kupfercitrat, Kupfertartrat, Kupferpantothenat, Kupferlactat, Kupfermalat, Kupfermandelat, Kupfersorbat Kupferbenzoat, Kupfersalicylat, Kupfergluconat, Kupferphytat, Kupferglycerophosphat und/oder Kupfercinnamat enthält.

8. Zahnpasta nach einem oder mehreren der vorhergehenden Ansprüche, dadurch gekennzeichnet, daß sie die Kupferverbindung in einer Menge von 0,05 bis 0,5 Gew.-% (berechnet als Cu), bezogen auf die Gesamtzusammensetzung, enthält.

## Claims

1. Aqueous toothpaste composition containing usual compounds, at least one polishing agent and 0,001 to 5% by weight of the total composition (calculated on Cu) of at least one copper ions releasing compound, characterized in that more than fifty percent of the polishing agent are composed of silica.

2. Toothpaste according to claim 1, characterized in that the silica polishing agent is composed at least partially of precipitated, optionally dehydrated, silica-gel.

3. Toothpaste according to claim 1, characterized in that the silica polishing agent is composed at least partially of silica-Xerogel.

4. Toothpaste according to claim 1, characterized in that the silica polishing agent is composed at least partially of, optionally partially dehydrated, silica-hydrogel.

5. Toothpaste according to one of the preceding claims, characterized in that it contains as copper ions releasing compound a water-soluble inorganic copper salt.

6. Toothpaste according to one of the claims 1 to 4, containing as copper ions releasing compound a copper salt of an organic acid.

7. Toothpaste according to claim 6, containing as copper salt of an organic acid at least one of the compounds copper citrate, copper tartrate, copper pantothenate, copper lactate, copper malate, copper mandelate, copper sorbate, copper benzoate, copper salicylate, copper gluconate, copper phytate, copper glycerophosphate and/or copper cinnamate.

8. Toothpaste according to one or more of the preceding claims, characterized in that it contains 0,05 to 0,5% by weight of the total composition (calculated on Cu) of the copper compound.

## Revendications

1. Composition de pâte dentifrice aqueuse contenant des matières habituelles, au moins un agent de polissage et 0,001 à 5% de poids (calculé comme Cu) d'au moins un composé livrant des ions de cuivre, caractérisé par le fait que plus que 50% de l'agent de polissage se composent de silice.

2. Pâte dentifrice selon revendication 1, caractérisée par le fait que l'agent de polissage de silice se compose au moins partiellement d'un gel de silice précipité, éventuellement déhydraté totalement ou partiellement.

3. Pâte dentifrice selon revendication 1, caractérisée par le fait que l'agent de polissage de silice se compose partiellement d'un xerogel de silice.

4. Pâte dentifrice selon revendication 1, caractérisée par le fait que l'agent de polissage de silice se compose au moins partiellement d'un hydrogel de silice, le cas échéant partiellement déhydraté.

5. Pâte dentifrice selon une des revendications précédentes, caractérisée par le fait qu'elle contient comme un composé livrant des ions de cuivre du sel de cuivre inorganique soluble dans l'eau.

6. Pâte dentifrice selon une des revendications 1à 4, caractérisée par le fait qu'elle contient comme un composé livrant des ions de cuivre du sel de cuivre d'une acide organique.

7. Pâte dentifrice selon revendication 6, caractérisée par le fait qu'elle contient comme sel de cuivre d'une acide organique au moins un des composés citrate de cuivre, tartrate de cuivre, pantothénate de cuivre, lactate de cuivre, malate de cuivre, mandélate de cuivre, sorbate de cuivre, benzoate de cuivre, salicylate de cuivre, gluconate de cuivre, phytate de cuivre, glycerophosphate de cuivre et/ou cinnamate de cuivre.

8. Pâte dentifrice selon une ou plusieurs des revendications précédentes, caractérisée par le fait qu'elle contient de 0,05 à 0,5% de poids de la composition totale (calculé comme Cu) du composé de cuivre.